# EUROPEAN PATENT APPLICATION

(11) **EP 3 639 883 A1**
(43) Date of publication of application: **22.04.2020**
(21) Application number: 18201148.6
(22) Date of filing: 18.10.2018
(51) Int. Cl.: A61N 1/04, A61N 1/36

(54) **KILOHERTZ E-TNS STIMULATION**

(71) Applicant: CEFALY Technology Sprl, 4102 Seraing (BE)
(72) Inventor: Rigaux, Pierre, 4020 Liège (BE)
(74) Representative: Brantsandpatents bvba

(57) **Abstract**

The current invention concerns a device and a method for the abortive or preventive treatment of a headache via external trigeminal nerve stimulation, in particular transcutaneous non-invasive electrical trigeminal stimulation via consecutive pulses. The pulses comprise a pulse repetition frequency of between 0.5 and 50 kilohertz; a pulse width of between 1 and 500 microseconds; and a pulse amplitude of between 0.1 and 200 milliampere.

## Description

### Technical field

The invention pertains to the technical field of devices and methods for the abortive or preventive treatment of a headache via transcutaneous electrical nerve stimulation (TENS), in particular via external trigeminal nerve stimulation (e-TNS).

### Background

Headaches, and in particular migraines, can significantly impair quality of life. Acute treatments are primarily pharmacologic approaches, with the most commonly used medications being analgesics, non-steroidal anti-inflammatory drugs (NSAIDs) and triptans. These drugs bear several contraindications and are associated with moderate to severe side effects. Excessive consumption may lead to headache chronification, medication overuse headache and/or resistance.

Neurostimulation has been shown to be a good alternative to pharmacologic approaches, and is suitable for both acute treatment and prevention treatment.

WO 2016/155 773 discloses a device for the transcutaneous electrical stimulation of the trigeminal nerve, and in particular the supratrochlear and supraorbital nerves of the ophthalmic branch of the trigeminal nerve. The device is configured for generating rectangular biphasic pulses with a width between 10 and 1000 µs, a frequency between 10 and 300 Hz, and an intensity between 1 and 35 mA. Preferential parameters are a width of about 250 µs, a frequency of about 120 Hz, and an intensity of about 16 mA.

Through its action on the nerve, e-TNS generates paresthesia on the forehead and/or the skull that can be felt as unpleasant by some patients. In addition, a portion of migraine patients suffer from forehead cutaneous allodynia. In combination with the forehead cutaneous allodynia, the paresthesia induced by e-TNS are very painful, which renders e-TNS treatment impossible for these patients.

Solomon and Guglielmo, "Treatment of Headache by Transcutaneous Electrical Stimulation", Headache 25(1), pages 12-15 (1985), doi:10.1111/j.1526-4610.1985.hed2501012.x discloses TENS via monophasic pulses with amperages of max. 4 mA, frequencies of 12 kHz to 20 kHz, and widths of approximately 30 µs. The document remains silent about trigeminal nerve stimulation.

US 2010/0 030 299 A1 discloses a battery-operated TENS unit to treat headache in an abortive and/or preventive manner. The document discloses comb-like electrodes to be placed at the back of the head. The document discloses, amongst other combinations, monophasic pulses of frequency/width: 3 kHz/50 µs and 10 kHz/40 µs. The document further discloses, amongst other examples, spaced rectangular biphasic pulse streams with frequency 1 kHz and a biphasic pulse width of 275 or 475 µs. The output current may be limited via a series resistance to a maximum of, amongst other values, 10 mA or 5 mA. The device according to the document is not suited for trigeminal nerve stimulation.

The present invention aims to resolve at least some of the problems mentioned above.

### Summary of the invention

In a first aspect, the present invention provides for a device for the abortive or preventive treatment of a headache via external trigeminal nerve stimulation (e-TNS), according to claim 1.

In a second aspect, the present invention provides for a method for the abortive or preventive treatment of a headache via e-TNS, according to claim 16.

In a third aspect, the present invention provides for use of a device according to the first aspect for the electrotherapeutic treatment of a neurological disorder, according to claim 18.

The present invention is advantageous, as the particular treatment parameters avoid or mitigate paresthesia.

### Description of figures

**Figures 1 to 5** show several embodiments of consecutive pulses.
**Figures 6A** **and** **6B** show an embodiment of a powering apparatus of a device according to the present invention.
**Figure 7** shows an embodiment of an electrode piece of a device according to the present invention applied to a person's forehead in the supraorbital region.
**Figure 8** shows a self-supporting device attached to a person's forehead, according to the embodiments of Figures 6A, 6B and 7.

### Detailed description of the invention

The present invention concerns a device and a method for the abortive or preventive treatment of a headache via external trigeminal nerve stimulation (e-TNS), as well as use of the device for the electrotherapeutic treatment of a neurological disorder.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specify the presence of what follows e.g. component and do not exclude or preclude the presence of additional, non-recited components, features, elements, members, steps, known in the art or disclosed therein.

The recitation of numerical ranges by endpoints includes all numbers and fractions subsumed within that range, as well as the recited endpoints.

In a first aspect, the invention provides a device for the abortive or preventive treatment of a headache via e-TNS. The device is configured for non-invasive transcutaneous electrical nerve stimulation (TENS) of the trigeminal nerve via consecutive pulses. The consecutive pulses comprise:
- a pulse repetition frequency of between 0.5 and 50 kilohertz;
- a pulse width of between 1 and 500 microseconds;
- a pulse amplitude of between 0.1 and 200 milliampere.

In a second aspect, the invention provides a method for the abortive or preventive treatment of a headache via e-TNS. The method comprises the step of non-invasive TENS of the trigeminal nerve via consecutive pulses. The consecutive pulses comprise:
- a pulse repetition frequency of between 0.5 and 50 kilohertz;
- a pulse width of between 1 and 500 microseconds;
- a pulse amplitude of between 0.1 and 200 milliampere.

The device according to the first aspect may be used in the preventive or abortive electrotherapeutic treatment of a headache. The invention may therefore also pertain to a device according to the first aspect **for use in** the preventive or abortive electrotherapeutic treatment of a headache.

The device according to the first aspect may be used in the electrotherapeutic treatment of a neurological disorder selected from the group consisting of migraine, tension headaches, cluster headaches, hemicrania continua, short-lasting unilateral neuralgiform headache attacks with conjunctival injection and tearing (SUNCT), chronic paroxysmal hemicrania, trigeminal neuralgia, facial nerve disturbances, fibromyalgia, chronic pain, depression, cyclothymia, post-traumatic stress syndrome, post-concussion syndrome, coma, anxiety, tremor, aphasia, obsessive compulsive disorder, insomnia, sleep disorders, sleep apnea syndrome, hypersomnia, epilepsy, drop attacks, attention deficit hyperactivity disorder, Parkinson's disease, Alzheimer's disease, multiple sclerosis, stroke and Cerebellar syndrome. The invention may therefore also pertain to a device according to the first aspect for use in the electrotherapeutic treatment of a neurological disorder selected from said group. The invention may also pertain to use of a device according to the first aspect for the electrotherapeutic treatment of a neurological disorder selected from said group.

e-TNS has previously been shown to be very effective for treatment of a headache, and in particular migraine. The treatment can be effected via non-invasive TENS, which, as opposed to percutaneous methods requiring electrode insertion, can be effected entirely non-invasive, i.e. without surgery. The applicant has surprisingly discovered that the particular treatment parameters of the present invention avoid or mitigate paresthesia during the treatment. The applicant deems this may be attributed to the following explanation. After the action potential, the membrane pumps modify the ions (Na+ and K+) permeability through the nerve membrane to reestablish the rest potential that is needed to make the nerve cell excitable again. When the repetition frequency of the impulses is high enough, the impulse is fired during the refractory period after the action potential and cannot generate a new action potential, but is enough to keep the cell depolarized and prevent the reestablishment of the rest polarization, making the nerve cell not able to trigger the action potential and paresthesia.

One of ordinary skill in the art will appreciate that a device according to the first aspect may be configured for carrying out a method according to the second aspect and that a method according to the second aspect may be performed with a device according to the first aspect. The different aspects of the present invention are hence interrelated. Therefore, each feature described above and below may relate to each of the aspects of the present invention, even if it has been described in conjunction with a particular aspect.

The "treatment parameters", as used herein, comprise properties of the consecutive pulses, such as pulse repetition frequency *f* and/or pulse repetition period *Tₚ*, pulse amplitude, pulse width *T_{w}*, and treatment time *Tₜ*. One of ordinary skill in the art appreciates that the pulse amplitude is the maximum absolute amplitude. The pulse repetition frequency is the inverse of the average pulse repetition period of the consecutive pulses.

In a preferred embodiment, the device and/or the method are configured for transcutaneous electrical nerve stimulation (TENS) of the supraorbital and supratrochlear nerves of the ophthalmic branch of the trigeminal nerve via the consecutive pulses. Preferably, the device and/or the method are configured for non-invasive TENS of the afferent paths of the supratrochlear and supraorbital nerves of the ophthalmic branch of the trigeminal nerve via the consecutive pulses. This is advantageous, as these nerves can be easily stimulated via non-invasive TENS through a skin portion, typically a person's forehead, and more specifically the supraorbital region, which is typically hairless or comprises a low amount of hair, allowing for easy application and removal of an electrode.

In a preferred embodiment, the pulses are rectangular pulses.

In a preferred embodiment, two consecutive pulses comprise a net charge transfer of at most 1 picocoulomb, preferably at most 0.1 picocoulomb, more preferably at most 0.01 picocoulomb, even more preferably at most 0.001 picocoulomb, and most preferably in essence no net charge transfer, i.e. a net charge transfer of in essence 0.0 picocoulomb.

In a preferred embodiment, a rectangular biphasic pulse comprises said two consecutive pulses, which are both rectangular and monophasic. The first pulse comprises an in essence constant first amplitude *A₁* during a first pulse width *T₁*. The second pulse, in time later than the first pulse, comprises an in essence constant second amplitude *A₂* during a second pulse width *T₂*. The second amplitude comprises an opposite sign with respect to the constant first amplitude, i.e. *A₁.A₂* < *0*. An in essence zero net charge transfer is realized when *A₁*.*T₁* + *A₂.T₂* ≈ 0. This is advantageous as the trigeminal nerve may be stimulated without net charge accumulation during treatment. Preferably, the biphasic pulse is a symmetric rectangular biphasic pulse. In that case, the first pulse width is in essence equal to the second pulse width, i.e. *T₁* ≈ *T₂,* and the constant first amplitude is in essence the opposite of the constant second amplitude, i.e. *A₁* + *A₂* ≈ *0*. The rectangular biphasic pulse may be gapped or may be non-gapped, i.e. the first pulse and the second pulse may or may not comprise a time gap in between. In the latter case, the second pulse is in essence immediately subsequent to the first pulse.

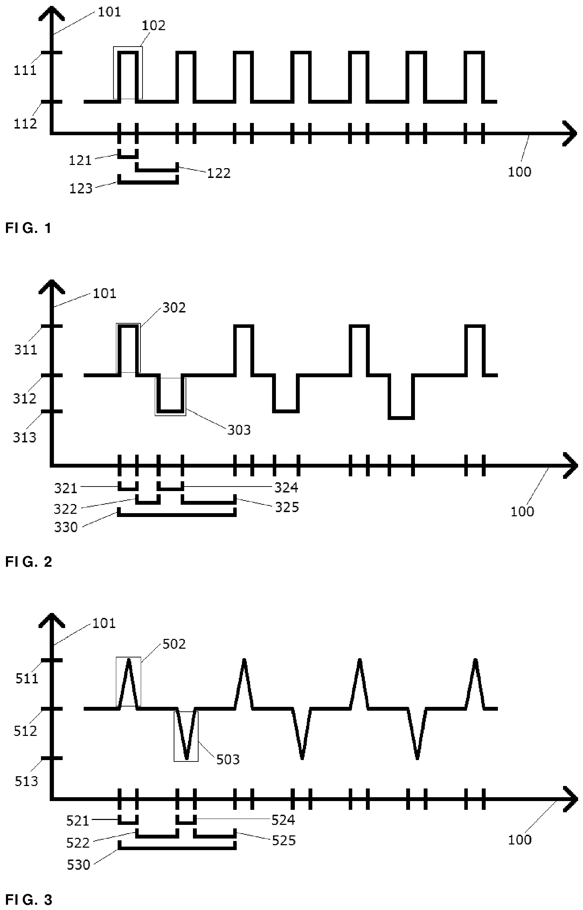

The non-limiting examples shown in Figures 1 to 4 illustrate several aspects of consecutive pulses, and are not intended to, nor should they be interpreted to, limit the scope of the invention.

Figure 1 shows several consecutive pulses on a two-dimensional graph comprising an amplitude axis (101) and a time axis (100). Amplitude (112) denotes that no current is transferred, i.e. an amplitude of 0.0 milliampere. A pulse (102) comprises a pulse width *T_{w}* (121) and a pulse repetition period *Tₚ* (123), both of which are time parameters. The difference *T_{g}* = *Tₚ* - *T_{w}* (122) between the pulse repetition period *Tₚ* (123) and the pulse width *T_{w}* (121) is the time gap after said pulse to the next pulse. The pulse repetition frequency can be calculated as *f* = *1* / *Tₚ.* The pulse furthermore comprises a pulse amplitude A (111). A pulse may be rectangular, thereby comprising an in essence constant amplitude A during the pulse width *T_{w},* as is the case in Figure 1. The applied current signal may be continuous. A pulse may thereby be triangular or sinusoidal, for example.

Figure 2 shows several consecutive pulses on a two-dimensional graph comprising an amplitude axis (101) and a time axis (100). Amplitude (312) denotes that no current is transferred, i.e. an amplitude of 0.0 milliampere. Two consecutive pulses (302, 303) comprise a net charge transfer of in essence 0.0 picocoulomb. A rectangular gapped biphasic pulse comprises a first monophasic rectangular pulse (302) and a second monophasic rectangular pulse (303). The first pulse (302) comprises a pulse width *T_{1,w}* (321), an amplitude *A₁* (311), and a time gap *T_{1,g}* (322) to the second pulse. The second pulse (303) comprises a pulse width *T_{2,w}* (324), and an amplitude *A₂* (313), whereby *A₁* and *A₂* comprise opposite phase, i.e. *A₁.A₂* < *0*. The second pulse comprises a time gap *T_{2,g}* (325) to the following pulse. The pulse repetition period *Tₚ* (330) is the repetition period of the biphasic pulse, i.e. the time span in between the beginning of two consecutive rectangular biphasic pulses. The pulse repetition frequency can be calculated as *f* = *1* / *Tₚ.* The two consecutive pulses (302, 303) comprises an in essence zero net charge transfer, i.e. *A₁.T_{1,w}* + *A₂.T_{2,w}* ≈ 0. The biphasic pulse may be symmetric, in which case *A₁* + *A₂* ≈ *0*, but may alternatively be non-symmetric, in which case |*A₁*| ≠ /*A₂*|.

Figure 3 shows several consecutive pulses on a two-dimensional graph comprising an amplitude axis (101) and a time axis (100). Amplitude (512) denotes that no current is transferred, i.e. an amplitude of 0.0 milliampere. Two consecutive triangular pulses (502, 503) comprise a net charge transfer of in essence 0.0 picocoulomb. A symmetric triangular gapped biphasic pulse comprises a first monophasic triangular pulse (502) and a second monophasic triangular pulse (503). The first pulse (502) comprises a pulse width *T_{1,w}* (521), an amplitude *A₁* (511), and a time gap *T_{1,g}* (522) to the second pulse. The second pulse (503) comprises a pulse width *T_{2,w}* (524), and an amplitude *A₂* (513), whereby *A₁* and *A₂* comprise opposite phase, i.e. *A₁.A₂* < *0*. The second pulse comprises a time gap *T_{2,g}* (525) to the following pulse. The pulse repetition period *Tₚ* (530) is the repetition period of the biphasic pulse, i.e. the time span in between the beginning of two consecutive biphasic pulses. The pulse repetition frequency can be calculated as *f* = *1* / *Tₚ.* The time gaps of the first pulse and the second pulse may or may not be equal. The pulse widths of the first pulse and the second pulse are equal, as the biphasic pulse is symmetric. The pulse amplitudes, i.e. the absolute values of the amplitudes, of the first pulse and the second pulse are equal, as the biphasic pulse is symmetric.

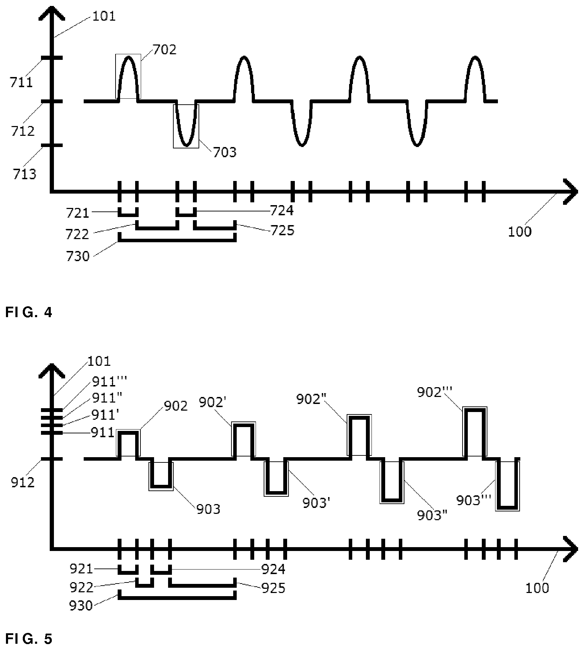

Figure 4 shows several consecutive pulses on a two-dimensional graph comprising an amplitude axis (101) and a time axis (100). Amplitude (712) denotes that no current is transferred, i.e. an amplitude of 0.0 milliampere. Two consecutive sinusoidal pulses (702, 703) comprise a net charge transfer of in essence 0.0 picocoulomb. A symmetric sinusoidal gapped biphasic pulse comprises a first monophasic sinusoidal pulse (702) and a second monophasic sinusoidal pulse (703). The first pulse (702) comprises a pulse width *T_{1,w}* (721), an amplitude *A₁* (711), and a time gap *T_{1,g}* (722) to the second pulse. The second pulse (703) comprises a pulse width *T_{2,w}* (724), and an amplitude *A₂* (713), whereby *A₁* and *A₂* comprise opposite phase, i.e. *A₁.A₂* < *0*. The second pulse comprises a time gap *T_{2,g}* (725) to the following pulse. The pulse repetition period *Tₚ* (730) is the repetition period of the biphasic pulse, i.e. the time span in between the beginning of two consecutive biphasic pulses. The pulse repetition frequency can be calculated as *f* = *1* / *Tₚ.* The time gaps of the first pulse and the second pulse may or may not be equal. The pulse widths of the first pulse and the second pulse are equal, as the biphasic pulse is symmetric. The pulse amplitudes, i.e. the absolute values of the amplitudes, of the first pulse and the second pulse are equal, as the biphasic pulse is symmetric.

In a preferred embodiment, the pulse repetition frequency is between 2 and 40 kilohertz. The pulse repetition frequency is preferably at least 4 kilohertz, more preferably at least 6 kilohertz, even more preferably at least 7 kilohertz, yet even more preferably at least 8 kilohertz, and most preferably at least 9 kilohertz. The pulse repetition frequency is preferably is preferably at most 30 kilohertz, more preferably at most 24 kilohertz, even more preferably at most 20 kilohertz, yet even more preferably at most 16 kilohertz, with greater preference at most 14 kilohertz, with an even greater preference at most 12 kilohertz, and with greatest preference at most 11 kilohertz. In a particularly preferred embodiment, the pulse repetition frequency is about 10 kilohertz.

In a preferred embodiment, the pulse width is between 4 and 240 microseconds, preferably between 8 and 120 microseconds, more preferably between 12 and 60 microseconds, even more preferably between 16 and 50 microseconds, yet even more preferably between 20 and 40 microseconds, and most preferably between 25 and 35 microseconds. In a particularly preferred embodiment, the pulse width is about 30 microseconds.

In a preferred embodiment, the pulse amplitude is between 1 and 128 milliampere, preferably between 2 and 64 milliampere, more preferably between 4 and 32 milliampere, even more preferably between 5 and 16 milliampere, most preferably between 6 and 12 milliampere. In a particularly preferred embodiment, the pulse amplitude is about 8 milliampere.

In a preferred embodiment, the treatment time is at least 5 minutes, preferably at least 10 minutes, more preferably at least 15 minutes, even more preferably at least 20 minutes, yet even more preferably at least 25 minutes, with greater preference at least 30 minutes, with even greater preference at least 40 minutes, with yet even greater preference at least 45 minutes, and with greatest preference at least 60 minutes.

In a preferred embodiment, the device according to first aspect and the method according to the second aspect may be configured for non-invasive TENS of the afferent paths of the supratrochlear and supraorbital nerves of the ophthalmic branch of the trigeminal nerve via rectangular pulses comprising:
- a pulse repetition frequency of between 8 and 12 kilohertz;
- a pulse width of between 12 and 60 microseconds;
- a pulse amplitude of between 5 and 16 milliampere; and
preferably during a treatment time of at least 5 minutes, and wherein preferably two consecutive pulses comprise a net charge transfer of at most 0.1 picocoulomb.

In a preferred embodiment, the device is configured for increasing, preferably linearly increasing, the pulse amplitude at a rate of at most 480 microampere/second prior to the treatment. In a preferred embodiment, the method comprises the step of increasing, preferably linearly increasing, the pulse amplitude at a rate of at most 480 microampere/second prior to the treatment step. The rate is preferably at most 240 microampere/second, more preferably at most 120 microampere/second, even more preferably at most 60 microampere/second, yet even more preferably at most 30 microampere/second, and most preferably at most 15 microampere/second. In a particularly preferred embodiment, the rate is about 9.5 microampere/second. Preferably, the increasing is performed during a start-up time span of at least 4 minutes, preferably at least 6 minutes, more preferably at least 8 minutes, even more preferably at least 10 minutes, and most preferably at least 12 minutes. Subsequently, the pulse amplitude remains in essence constant during the treatment time. This is advantageous, as it allows a person to become gradually accustomed to the amplitude of the treatment during a start-up phase prior to the treatment.

The non-limiting example shown in Figure 5 illustrates several aspects of the start-up, and is not intended to, nor should it be interpreted to, limit the scope of the invention.

Figure 5 shows several consecutive symmetric biphasic rectangular pulses during the start-up on a two-dimensional graph comprising an amplitude axis (101) and a time axis (100). Amplitude (912) denotes that no current is transferred, i.e. an amplitude of 0.0 milliampere. Each biphasic pulse comprises a first pulse (902, 902', 902", 902"') and a second pulse (903, 903', 903", 903*'"*), which comprise successively larger pulse amplitudes (911, 911', 911", 911*'"*) over the subsequent biphasic pulses. The increase of the pulse amplitude is in the present case linear. The first pulse (902) comprises a pulse width *T_{1,w}* (921), and a time gap *T*_{*1*,*g*} (922) to the second pulse. The second pulse (903) comprises a pulse width *T_{2,w}* (924), and a time gap *T_{2,g}* (925) to the following pulse. The pulse frequency can be calculated as *f* = *2* / (*T_{1,p}* + *T_{2,p}*). The time gaps of the first pulse and the second pulse may or may not be equal. The pulse widths of the first pulse and the second pulse are equal, as the biphasic pulses are symmetric. The pulse repetition period *Tₚ* (930) is the repetition period of the biphasic pulse, i.e. the time span in between the beginning of two consecutive rectangular biphasic pulses. The pulse repetition frequency can be calculated as *f* = *1* / *Tₚ.*

In a preferred embodiment, the device is portable and configured for self-support on a person's forehead. The portable device may comprise an electrode piece and a powering apparatus. The powering apparatus may be configured for manual attachment to and manual detachment from the electrode piece. The electrode piece may comprise a pair of spaced self-adhesive conductive gel zones for application to a person's forehead. Preferably, the powering apparatus is configured for manual attachment to and manual detachment from the electrode piece via at least two engaging pairs of metallic contacts. The engaging pairs of metallic contacts are thereby further configured for establishing electrical connection between the powering apparatus and the pair of spaced self-adhesive conductive gel zones. Preferably, an engaging pair of metallic contacts comprises a snap button or a magnet. Preferably, the electrode piece is configured for application on the person's forehead in the supraorbital region, thereby covering the afferent paths of the supratrochlear and supraorbital nerves of the ophthalmic branch of the trigeminal nerve.

In a preferred embodiment, the device comprises a powering apparatus configured for determining an impedance for electrical current transfer, whereby the powering apparatus is further configured for aborting treatment upon determining an impedance above a predefined threshold.

In a preferred embodiment, the device comprises at least one physiological sensor. The device is configured for aborting treatment and/or adapting one or more treatment parameters based at least in part on a signal obtained from said at least one physiological sensor.

In a preferred embodiment, the device comprises a powering apparatus configured for retaining a battery. The powering apparatus may retain a battery. The battery may be rechargeable. The powering apparatus may comprise a USB port for recharging the battery. The USB port may also allow for transferring treatment parameters to the powering apparatus and/or receiving information about a treatment session from the powering apparatus. The powering apparatus may comprise a contactless recharging module for inductively or capacitively recharging the battery.

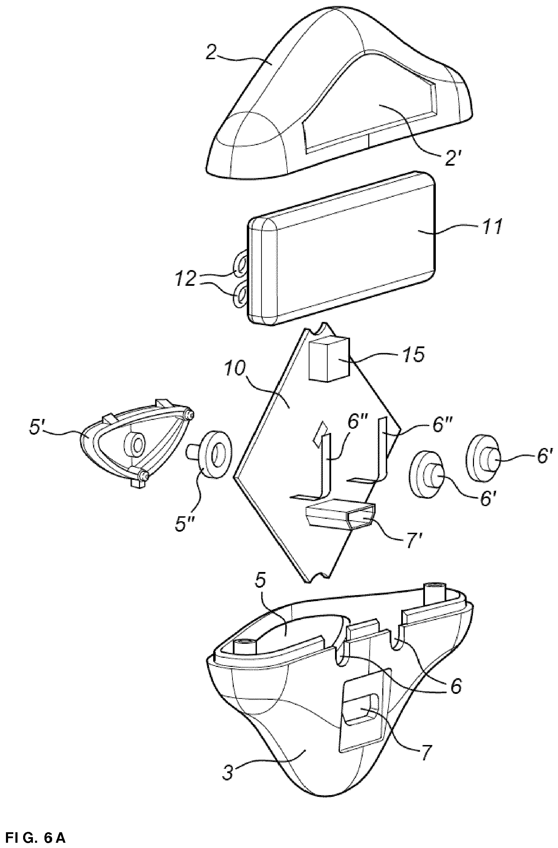

The non-limiting examples shown in Figures 6A to 8 illustrate several mechanical aspects of an embodiment of a device according to the present invention, and are not intended to, nor should they be interpreted to, limit the scope of the invention.

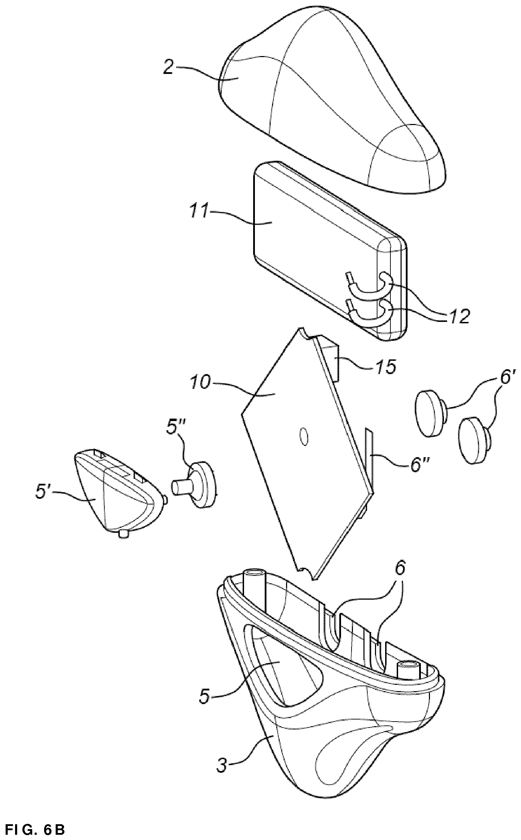

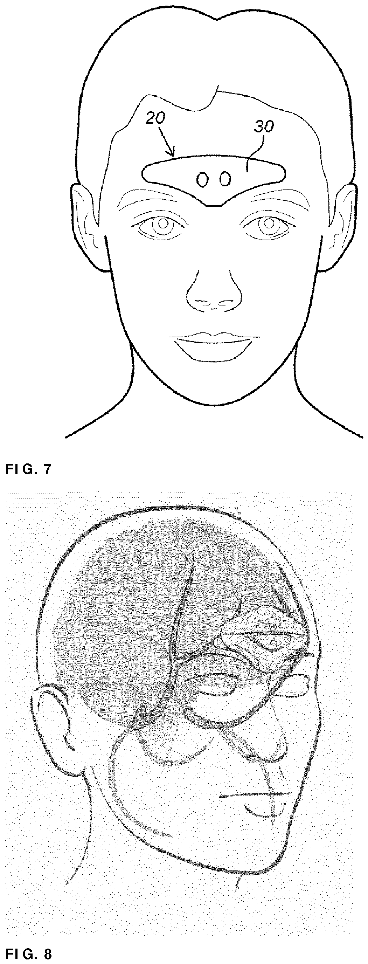

Figures 6A and 6B show an embodiment of a powering apparatus of a device according to the present invention. Figure 7 shows an embodiment of an electrode piece (30) of a device according to the present invention, applied to a person's forehead in the supraorbital region (20). Figure 8 shows a self-supporting device attached to a person's forehead, comprising a powering apparatus according to Figures 6A and 6B and an electrode piece according to Figure 7.

The powering apparatus comprises an upper part (2) and a lower part (3) which may be relatively fixed. The lower part (3) comprises a control opening (5) through which at least one control means (5'), such as a button for example, may protrude. The lower part (3) furthermore comprises two magnet openings (6), through each of which a magnet (6') may protrude. The lower part (3) furthermore also comprises a micro-USB opening (7) through which a micro-USB port (7') may protrude.

The electrode piece (30) comprises a front side and a back side. At the back side (not shown), the electrode piece (30) comprises a pair of spaced self-adhesive conductive gel zones for application to a person's forehead. At the front side, the electrode piece (30) comprises a pair of metallic contacts, preferably magnetic metallic contacts, comprising a relative distance corresponding to the magnet openings (6) of the powering apparatus. Each metallic contact is electrically connected to a self-adhesive conductive gel zone. The device is via the magnets and metallic contacts configured for removable attachment of the powering apparatus to the electrode piece.

The upper and lower parts (2, 3) provide for an internal space for housing a printed circuit board (PCB) (10) and a battery (11). Mechanical movement of the control means (5') may be transferred via an intermediary component (5") to the PCB (10), where said mechanical movement may be converted to suitable impedance (resistance, capacitance, inductance), voltage and/or current changes. The battery (11) may be connected via at least two wires (12) to the PCB (10). The PCB comprises two contact blades (6") configured for retaining the battery against the PCB and further each configured for contact with a magnet (6').

The PCB further comprises a microcontroller unit (15) and a tangible non-transitory computer-readable storage medium. The micro-USB port allows for recharging the battery and for transferring treatment parameters and/or other information to and/or from the powering device.

The PCB is configured for:
- determining an impedance between the blades (6") or between the pair of self-adhesive conductive gel zones; and
- upon determining an impedance outside a predefined region of interest (e.g. a resistance above a predefined threshold) during start-up or treatment, aborting the treatment.

## Claims

1. Device for the abortive or preventive treatment of a headache via external trigeminal nerve stimulation, the device configured for transcutaneous non-invasive electrical trigeminal nerve stimulation via consecutive pulses comprising:
- a pulse repetition frequency of between 0.5 and 50 kilohertz;
- a pulse width of between 1 and 500 microseconds; and
- a pulse amplitude of between 0.1 and 200 milliampere.

2. Device according to any one of the preceding claims, wherein the device is configured for transcutaneous non-invasive electrical nerve stimulation of the afferent paths of the supratrochlear and supraorbital nerves of the ophthalmic branch of the trigeminal nerve via said pulses.

3. Device according to any one of the preceding claims, wherein the pulses are rectangular pulses.

4. Device according to any one of the preceding claims, wherein two consecutive pulses comprise a net charge transfer of at most 1 picocoulomb, preferably at most 0.1 picocoulomb, more preferably at most 0.01 picocoulomb, even more preferably 0.001 picocoulomb, and most preferably in essence 0.0 picocoulomb.

5. Device according to any one of the preceding claims, wherein said pulse repetition frequency is between 2 and 40 kilohertz, wherein said pulse repetition frequency is preferably at least 4 kilohertz, more preferably at least 6 kilohertz, even more preferably at least 7 kilohertz, yet even more preferably at least 8 kilohertz, and most preferably at least 9 kilohertz, and wherein said pulse repetition frequency is preferably at most 30 kilohertz, more preferably at most 24 kilohertz, even more preferably at most 20 kilohertz, yet even more preferably at most 16 kilohertz, with greater preference at most 14 kilohertz, with an even greater preference at most 12 kilohertz, and with greatest preference at most 11 kilohertz.

6. Device according to any one of the preceding claims, wherein said pulse width is between 4 and 240 microseconds, preferably between 8 and 120 microseconds, more preferably between 12 and 60 microseconds, even more preferably between 16 and 50 microseconds, yet even more preferably between 20 and 40 microseconds, and most preferably between 25 and 35 microseconds.

7. Device according to any one of the preceding claims, wherein said pulse amplitude is between 1 and 128 milliampere, preferably between 2 and 64 milliampere, more preferably between 4 and 32 milliampere, even more preferably between 5 and 16 milliampere, most preferably between 6 and 12 milliampere.

8. Device according to any one of the preceding claims, wherein the consecutive pulses are applied during a treatment time of at least 5 minutes, preferably at least 10 minutes, more preferably at least 15 minutes, even more preferably at least 20 minutes, yet even more preferably at least 25 minutes, with greater preference at least 30 minutes, with even greater preference at least 40 minutes, with yet even greater preference at least 45 minutes, and with greatest preference at least 60 minutes.

9. Device, according to all of the preceding claims, for the abortive or preventive treatment of a headache via external trigeminal nerve stimulation, the device configured for transcutaneous non-invasive electrical nerve stimulation of the afferent paths of the supratrochlear and supraorbital nerves of the ophthalmic branch of the trigeminal nerve via rectangular pulses comprising:
- a pulse repetition frequency of between 8 and 12 kilohertz;
- a pulse width of between 12 and 60 microseconds; and
- a pulse amplitude of between 5 and 16 milliampere,
and wherein two consecutive pulses comprise a net charge transfer of at most 0.1 picocoulomb.

10. Device according to any one of the preceding claims, wherein the device is configured for increasing, preferably linearly increasing, the pulse amplitude at a rate of at most 480 microampere/second, preferably at most 240 microampere/second, more preferably at most 120 microampere/second, even more preferably at most 60 microampere/second, yet even more preferably at most 30 microampere/second, and most preferably at most 15 microampere/second, prior to said treatment.

11. Portable device according to any one of the preceding claims, comprising an electrode piece and a powering apparatus, the powering apparatus configured for manual attachment to and manual detachment from the electrode piece, whereby the electrode piece comprises a pair of spaced self-adhesive conductive gel zones for application to a person's forehead, whereby the device is configured for self-support on the person's forehead, preferably whereby the powering apparatus is configured for manual attachment to and manual detachment from the electrode piece via at least two engaging pairs of metallic contacts which are further configured for establishing electrical connection between the powering apparatus and the pair of spaced self-adhesive conductive gel zones, more preferably wherein an engaging pair of metallic contacts comprises a snap button or a magnet.

12. Portable device according to preceding claim 11, wherein the electrode piece is configured for application on the person's forehead in the supraorbital region, thereby covering the afferent paths of the supratrochlear and supraorbital nerves of the ophthalmic branch of the trigeminal nerve.

13. Device according to any one of the preceding claims, wherein the device comprises at least one physiological sensor, and whereby the device is configured for aborting treatment and/or adapting one or more treatment parameters based at least in part on a signal obtained from said at least one physiological sensor.

14. Device according to any one of the preceding claims **for use in** the preventive or abortive electrotherapeutic treatment of a headache.

15. Device according to any one of the preceding claims 1 to 13 **for use in** the electrotherapeutic treatment of a neurological disorder selected from the group consisting of migraine, tension headaches, cluster headaches, hemicrania continua, short-lasting unilateral neuralgiform headache attacks with conjunctival injection and tearing (SUNCT), chronic paroxysmal hemicrania, trigeminal neuralgia, facial nerve disturbances, fibromyalgia, chronic pain, depression, cyclothymia, post-traumatic stress syndrome, post-concussion syndrome, coma, anxiety, tremor, aphasia, obsessive compulsive disorder, insomnia, sleep disorders, sleep apnea syndrome, hypersomnia, epilepsy, drop attacks, attention deficit hyperactivity disorder, Parkinson's disease, Alzheimer's disease, multiple sclerosis, stroke and Cerebellar syndrome.

16. Method for the abortive or preventive treatment of a headache via external trigeminal nerve stimulation, the method comprising the step of transcutaneous non-invasive electrical trigeminal nerve stimulation, preferably transcutaneous non-invasive electrical nerve stimulation of the afferent paths of the supratrochlear and supraorbital nerves of the ophthalmic branch of the trigeminal nerve, via consecutive pulses, preferably rectangular pulses, comprising:
- a pulse repetition frequency of between 0.5 and 50 kilohertz, preferably of between 8 and 12 kilohertz;
- a pulse width of between 1 and 500 microseconds, preferably of between 12 and 60 microseconds; and
- a pulse amplitude of between 0.1 and 200 milliampere, preferably of between 5 and 16 milliampere,
and wherein preferably two consecutive pulses comprise a net charge transfer of at most 0.1 picocoulomb.

17. Method according to preceding claim 16, comprising the step of increasing, preferably linearly increasing, the pulse amplitude at a rate of at most 480 microampere/second, preferably at most 240 microampere/second, more preferably at most 120 microampere/second, even more preferably at most 60 microampere/second, yet even more preferably at most 30 microampere/second, and most preferably at most 15 microampere/second, prior to said step of said treatment.

18. Use of a device according to any one of the preceding claims 1 to 13 for the electrotherapeutic treatment of a neurological disorder selected from the group consisting of migraine, tension headaches, cluster headaches, hemicrania continua, short-lasting unilateral neuralgiform headache attacks with conjunctival injection and tearing (SUNCT), chronic paroxysmal hemicrania, trigeminal neuralgia, facial nerve disturbances, fibromyalgia, chronic pain, depression, cyclothymia, post-traumatic stress syndrome, post-concussion syndrome, coma, anxiety, tremor, aphasia, obsessive compulsive disorder, insomnia, sleep disorders, sleep apnea syndrome, hypersomnia, epilepsy, drop attacks, attention deficit hyperactivity disorder, Parkinson's disease, Alzheimer's disease, multiple sclerosis, stroke and Cerebellar syndrome.
